# EUROPEAN PATENT APPLICATION

(11) **EP 1 325 908 A1**
(43) Date of publication of application: **09.07.2003**
(21) Application number: 01000756.5
(22) Date of filing: 14.12.2001
(51) Int. Cl.: C07C 327/32

(54) **Process for the preparation of an enantiomerically enriched thio compound**

(71) Applicant: DSM N.V., 6411 TE Heerlen (NL)
(72) Inventor: Kuilman, Thijs, 5925 BA, Blerick (NL); Broxterman, Quirinus Bernardus, 6151 JA, Sittard (NL); Verzijl, Gerardus Karel Maria, 5855 AR, Well (NL); Straatman, Henricus Martinus Maria Gerardus, 5961 LG, Horst (NL)
(74) Representative: Jacobs, Monique S.N.

(57) **Abstract**

The invention relates to a process for the preparation of an enantiomerically enriched compound of formula 1 or a salt thereof, in which R¹ and R² each independently represent an (hetero)alkyl or (hetero)aryl group, wherein R¹-C(O)-SH or a salt thereof, with R¹ as defined above, is reacted with R²-C(C=CH₂)-C(O)OH with R² as defined above, wherein the reaction takes place in the presence of catalyst is an enantiomerically enriched Lewis acid containing a transition metal chosen from the group of Ti, Zr and Hf or a combination thereof and at least one enantiomerically enriched ligand, and in the presence of an alkali metal ion, an alkaline earth metal ion or an ammonium ion.

## Description

The invention relates to a process for the preparation of an enantiomerically enriched thio compound of formula 1 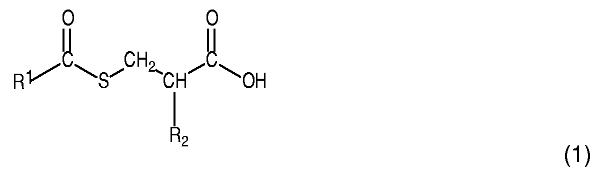 or a salt thereof, wherein R¹ and R² each independently represent a (hetero)alkyl or (hetero)aryl group, wherein a thio compound of formula 2 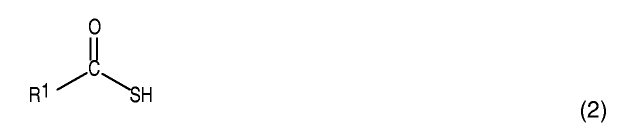 with R¹ as defined above, is reacted with an alkene of formula 3 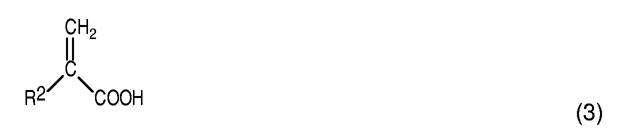 or a salt thereof, with R² as defined above.

Such a process is known from JP-A-9278746, which discloses the preparation of enantiomerically enriched 3-thioacyl-2-aralkyl propionic acid esters by contacting a thioacid with a 2-aralkyl acrylic acid ester in the presence of a cinchona alkaloid or a cinchona alkaloid whose OH is esterified.

The aim of the invention is to provide a commercially attractive alternative process comprising the preparation of an enantiomerically enriched compound of formula 1 by asymmetric catalysis.

This is achieved according to the invention by performing the reaction in the presence of an enantiomerically enriched Lewis acid containing a transition metal chosen from the group of Ti, Zr and Hf or a combination thereof and at least one enantiomerically enriched ligand, and in the presence of an alkali metal ion, an alkaline earth metal ion or an ammonium ion.

It is surprising that such a process results in the formation of an enantiomerically enriched product.

The terms Lewis acid and ligand are commonly known in the art and defined in for example "Advanced Inorganic Chemistry", F.A. Cotton and G. Wilkinson, John Wiley & Sons, 5^{th} ed., 1988, p. 35-36.

In the catalyst used in the process according to the invention the transition metal chosen from the group of Ti, Zr and Hf is preferably present as M(IV), in which M = Ti, Zr or Hf. As a source for the transition metal any salt or complex of the transition metal may be used. Examples of suitable salts or complexes are Ti halogenides, for example TiCl₄, and Ti alkoxides, for example Ti(OC₂H₅)₄, Ti(OCH(CH₃)₂)₄, Ti(O(CH₂)₃CH₃)₄ and Ti(OCH(C₂H₅)(C₄H₉))₄. The catalyst can suitably be prepared starting form a transition metal complex containing achiral ligands, many of which are readily available and relatively cheap. If such starting materials are used it may be desirable to remove at least part of the achiral ligands before said catalyst is applied in the process according to the invention to avoid competition between such achiral ligands and the enantiomerically enriched ligand for binding to the transition metal and to avoid the formation of an achiral Lewis acid which may have an adverse effect on the process. A broad range of separation techniques may be used to remove said ligands, for example distillation and extraction.

Preferably the catalyst contains Ti. It has been found that catalysts containing Ti and an enantiomerically enriched ligand possesses both a relatively high enantioselectivity and a high catalytic activity.

The amount of transition metal used can vary within wide ranges and is dependent on for instance the type of transition metal and enantiomerically enriched ligand, the reactants and the reaction conditions, for example the temperature, the concentration of the transition metal and the enantiomerically enriched ligand and the reactants and the type of solvent. Preferably 0.5-200 mol% of transition metal is used based on the amount of R²-C(=CH₂)-COOH or a salt thereof, more preferably 1-100 mol%, most preferably 2-40 mol%.

The enantiomerically enriched Lewis acid contains at least one enantiomerically enriched ligand with an *e.e.* of > 90 %, preferably > 95 %, more preferably > 98%. A wide range of such ligands may be used. Examples of suitable enantiomerically enriched ligands are (derivatives of) enantiomerically enriched alcohols. Preferably an enantiomerically enriched diol is present as the ligand, more preferably an enantiomerically enriched tartaric acid derivative, most preferably an enantiomerically enriched tartaric acid ester with formula R³OC(O)-CH₂OH-CH₂OH-C(O)OR⁴, R³ and R⁴ each independently representing a (hetero)alkyl or (hetero)aryl group having 1-50 carbon atoms. Enantiomerically enriched tartaric acid esters are commercially available or relatively easily synthesised. Examples of suitable enantiomerically enriched tartaric acid esters are for instance enantiomerically enriched diethyl tartrate, enantiomerically enriched di-*iso*-propyl tartrate, enantiomerically enriched di-*iso*-propoxyethyl tartrate, enantiomerically enriched di-2,2,2-trichloorethyl tartrate and enantiomerically enriched di-4-benzyloxybutyl tartrate.

The amount of enantiomerically enriched ligand relative to the amount of transition metal ion is not particularly critical. Preferably an approximately stoichiometric amount of enantiomerically enriched ligand is used based on the amount and valency of the transition metal ion. For example, if M(IV) is used in combination with a monodentate ligand, the ratio enantiomerically enriched ligand : M(IV) is preferably between 3 : 1 and 5 :1, more preferably between 3.5:1 and 4.5:1, most preferably between 3.8 :1 and 4.2 : 1, while in the case of a bidentate ligand, the ratio enantiomerically enriched ligand : M(IV) is preferably between 1.5 : 1 and 2.5 : 1, more preferably between 1.75 : 1 and 2.25 : 1, most preferably between 1.9:1 and 2.1 : 1.

The process according to the invention is carried out in the presence of an alkali metal ion, an alkaline earth metal ion or an ammonium ion, which may be introduced to the reaction mixture in various ways, for example by adding as a salt, preferably as a salt of one of the reagents, or by using a "solid carrier", for example an ion exchange resin, containing the ion. Preferably Na⁺ or Li⁺ are used, more preferably a Na or Li salt of one of the reagents. Herein reagent refers to either a thio compound according to formula 2 or an alkene according to formula 3.

Preferably between 0.1 and 50 mol% of alkali metal ion, alkaline earth metal ion or ammonium ion is used based on the amount of transition metal.

R¹ and R² each independently represent a (hetero)alkyl or (hetero)aryl group. Suitable choices for R¹ and R² are an optionally substituted (hetero)alkyl group, optionally containing for instance one or more N, O or S atoms, with for example 1 to 50 C atoms, for example a methyl, ethyl, propyl, butyl, tertiary butyl, benzyl group or an optionally substituted (hetero)aryl group with for example 1 to 50 C atoms, for example a phenyl, naftyl, pyridyl, pyrrolyl, chinolyl, isochinolyl, furyl, thienyl, benzofuryl, indenyl, pyrimidinyl, pyrazolyl or imidazolyl group. The (hetero)alkyl or (hetero)aryl group of R¹ or R² is optionally substituted with one or more substituents which are inert under the chosen reaction conditions. Suitable examples of such substituents are an (hetero)alkyl group with for example 1 to 20 C atoms, for example a methyl, ethyl, isobutyl or trifluoromethyl group; an alkenyl group with for example 2 to 20 C atoms, an alkoxy group with for example 1 to 20 C atoms, a (hetero)aryl group with for example 1 to 20 C atoms, an aryloxy group with for example 1 to 20 C atoms, an amino group, a hydroxy group or a halogen.

Particularly suitable examples of R¹ are a methyl group, a tertiary butyl group, a cyclohexyl group and a phenyl group.

Particularly suitable examples of R² are a methyl group; a substituted or unsubstituted benzyl group, for example a benzyl group, a 4-chlorobenzyl group, a 4-bromobenzyl group and a 4-methoxybenzyl group; a 1-naphtalylmethyl group; a 2-naphtalylmethyl group; a 1,1'-biphenylmethyl group; a substituted or unsubstituted aryloxyalkyl group, for example a substituted or unsubstituted phenoxyalkyl group, for example a 3-(4-ethylphenoxy)propyl group, a 3-(4-benzoylphenoxy)propyl group, a 3-(4-methylphenoxy)propyl group, a 3-(3-ethylphenoxy)propyl group, a 3-(4-(phenylmethyl)phenoxy)propyl group, a 4-(3-cyanophenoxy)butyl group, a 4-(4-phenoxyphenoxy)butyl group, a 4-(2,6-dimethylphenoxyl)butyl group, a 4-(4-n-propoxyphenoxy)butyl group, a 4-(4-iodophenoxy)butyl group, a 4-[4-(acetylamino)phenoxy]butyl group, a 4-[4-(phenoxymethyl)phenoxy]butyl group, a 4-[4-(trifluoromethyl)phenoxy]butyl group, a 4-(3-ethylphenoxy)butyl group, a 4-(3-chlorophenoxy)butyl group, a 5-[4-(phenoxymethyl)phenoxy]pentyl group, a 5-(4-ethoxyphenoxy)pentyl group and a 6-phenylhexyl group; a substituted or unsubstituted phenylcarbonylmethyl group, for example a (4-bromophenyl)carbonylmethyl group, a (3,4-dimethylphenyl)carbonylmethyl group, a (2,4-dimethylphenyl)carbonylmethyl group, a [4-(4-chlorophenoxy)phenyl]carbonylmethyl group, a (4-phenoxyphenyl)carbonylmethyl group, a (4-methoxyphenyl)carbonylmethyl group, a (4-isopropoxyphenyl)carbonylmethyl group, a (4-methylphenyl)carbonylmethyl group, a (4-bromophenyl)carbonylmethyl group, a (4-chlorophenyl)carbonylmethyl group and a (4-fluorophenyl)carbonylmethyl group; a substituted or unsubstituted pyridylmethyl group, for example a (2-aminopyridin-5-yl)methyl group, a [2-(t-butoxycarbonylamino)pyridin-5-yl]methyl group and a [2-(t-butoxycarbonylamino)-6-methyl-pyridin-5-yl]methyl group.

The compound of formula (3) may be used as a carboxylic acid or a carboxylic acid salt, for example an (alkali or alkaline earth) metal or ammonium salt. Preferably a carboxylic acid or a Na, K, Li salt thereof are used, more preferably a carboxylic acid is used. The compound of formula (3) may be formed *in situ* using a precursor that is capable of forming such compound under the applied reaction conditions.

The process according to the invention is particularly suitable for the production of carboxylic acids according to formula 1 in which R¹ represents a methyl group and R² represents a benzyl or a methyl group.

The molar ratio between the reactants R¹-C(O)-SH and R²-C(=CH₂)-C(O)OH or a salt thereof is not particularly critical and may vary between for example 10:1 and 1:10, preferably between 5:1 and 1:5, more preferably between 2.5:1 and 1:2.5, most preferably between 1.6:1 and 1:1.1.

The temperature at which the process is performed is not particularly critical and can easily be optimised by the skilled person. Usually a reaction temperature between 0 and 150°C is applied, preferably between 20 and 110°C, more preferably between 30 and 80°C. Surprisingly it was found that in some cases at a relatively high temperature both the output and enantioselectivity were higher.

The process according to the invention preferably is carried out in the presence of a solvent. In the case of one or more liquid reagents the process can also be carried out in the absence of a solvent. Preferably a solvent is applied. A wide range of organic solvents can be used in the process, as long as they are inert under the chosen reaction conditions, for example optionally substituted aliphatic hydrocarbons, for instance n-hexane, n-heptane, dichloromethane, 1,2-dichloroethane and trichloromethane, optionally substituted (hetero)aromatic hydrocarbons, for instance toluene or N-methyl pyrrolidon, optionally substituted ethers, for instance methyl t-butyl ether or tetrahydrofuran, optionally substituted carboxylic acids, for instance acetic acid, optionally substituted esters, for instance ethyl acetate, optionally substituted nitriles, for instance acetonitrile or optionally substituted ketones, for instance methyl isobutyl ketone. Some of these solvents were found to have a positive effect on the selectivity of the reaction and thus on the *ee* of the product, the effect depending on e.g. the reactants and catalyst applied. Preferably an optionally substituted alifatic or aromatic hydrocarbon or an optionally substituted ether is used as the solvent, more preferably toluene.

The enantiomerically enriched thio compound prepared by the method according to the invention can be isolated using various methods. Suitable methods are, for instance, isolation as the compound as such, for example by crystallisation, conversion of the compound with an achiral base into a salt and subsequent crystallisation of said salt or reacting the compound with an enantiomerically enriched base and subsequent crystallisation of the formed diastereomeric salt.

In one embodiment of the invention the enantiomerically enriched thio compound prepared by the method according to the invention is isolated by contacting with an amine, followed by crystallisation of the formed salt of the enantiomerically enriched thio compound and the amine and conversion of the salt into the enantiomerically enriched thio compound and the amine using methods known *per se*. It was found that by applying such procedure the transition metal can be separated from the enantiomerically enriched thio compound and that the enantiomerically enriched thio compound can be obtained in high purity. Different types of amines can be used, both aliphatic and aromatic amines, the optimal choice of the amine depending on the compound to be isolated. For example, it has been found that (S)-3-thioacetyl-2-benzylpropionic acid can suitably isolated in the presence of cyclohexylamine.

The invention will now be explained in more detail with reference to the following examples, without however being limited thereto.

### Examples

### Example I: preparation of the catalyst

To 200 ml of toluene, 18.72 g (66 mmol) of titanium tetra-*iso*-propoxide and 28.32 g (137 mmol) of L-diethyl tartrate were added at 20°C under nitrogen atmosphere. The mixture was stirred for 48 hours at 20°C. After removal of the volatiles by distillation at reduced pressure (10 mg Hg) and elevated temperature (60ºC) an oil was obtained, which was dissolved in toluene to a total volume of 200 ml. The resulting catalyst solution, containing 0.33 mol Ti per liter, was used in examples II-XIV.

### Example II: preparation of enantiomerically enriched (S)-3-thioacetyl-2-benzyl propionic acid

To 90 ml of toluene, 110 mg (1 mmol) of sodium methacrylate and 3.24 g (20 mmol) of 2-benzylacrylic acid were added at 70°C. The resulting mixture was stirred for 15 minutes, followed by the addition of 8.5 ml of the catalyst solution prepared in example 1 (2.8 mmol Ti-catalyst) at 70°C. The mixture was stirred for an additional 15 minutes at 70°C. 1.56 ml (22.7 mmol) of thioacetic acid was dosed to the reaction mixture at 70°C within 15 minutes. The reaction mixture was stirred for 1 hour at 70°C to complete the conversion. The *e.e.* of (S)-3-thioacetyl-2-benzylpropionic acid in the reaction mixture, as determined by HPLC, was 44%.

### Example III: preparation of enantiomerically enriched (S)-3-thioacetyl-2-benzyl propionic acid

To 700 ml toluene, 4.93 g (26.8 mmol) of sodium 2-benzylacrylate and 24.0 g (148 mmol) of 2-benzylacrylic acid were added at 70°C. The resulting mixture was stirred for 15 minutes to dissolve most of the 2-benzylacrylic acid. 200 ml of the catalyst solution prepared in example 1 (66 mmol Ti-catalyst) was added at 70°C. The mixture was stirred for an additional 15 minutes at 70°C. A solution of 18 ml of thioacetic acid (262 mmol) in 50 ml toluene was dosed to the reaction mixture at 70°C within 30 minutes. The reaction mixture was stirred for 3 hours at 70°C to complete the conversion. The e.e. of S-3-thioacetyl-2-benzyl propionic acid in the reaction mixture, as determined by HPLC, was 50%. The reaction mixture was subsequently cooled to 35°C, after which the excess of thioacetic acid was removed by distillation at reduced pressure at 35°C until the total weight of the residue was 350 gram. After further cooling the mixture to 25°C, 300 ml of toluene was added, followed by the addition of 20 grams of cyclohexylamine within 30 minutes, during which the product started to crystallise. The heterogeneous mixture was stirred at 25°C for an additional 2 hours. The solid was collected by filtration and washed with 250 ml of toluene. The wet cake was air dried at room temperature, yielding 46.4 g of the salt of (S)-3-thioacetyl-2-benzyl propionic acid and cyclohexylamine in a purity of > 98%, according to HPLC. The e.e. of the (S)-3-thioacetyl-2-benzyl propionic acid in the salt was 55%, which is higher than the e.e. before contacting with cyclohexylamine. The overall yield based on the amount of sodium 2-benzylacrylate and 2-benzylacrylic acid was 78%.

### Example IV: preparation of enantiomerically enriched (S)-3-thioacetyl-2-methyl propionic acid

To 95 ml of toluene, 40.1 mg (0.37 mmol) of sodium methacrylate and 1.72 g (20 mmol) of methacrylic acid were added at 70°C. The resulting mixture was stirred for 15 minutes. 3 ml of the catalyst solution prepared in example 1(1 mmol of Ti-catalyst) was added at 70°C. After addition of the catalyst the mixture was stirred for an additional 15 minutes at 70°C. 1.56 ml (22.7 mmol) of thioacetic acid was dosed to the reaction mixture at 70°C within 15 minutes. The reaction mixture was stirred for 1 hour at 70°C to complete the conversion. The *e.e.* of (S)-3-thioacetyl-2-methylpropionic acid in the reaction mixture, as determined by HPLC, was 44%.

### Example V: preparation of enantiomerically enriched (S)-3-thioacetyl-2-methyl propionic acid

To 95 ml of toluene, 82 mg (0.76 mmol) of sodium methacrylate and 1.72 g (20 mmol) of methacrylic acid were added at 70°C. The resulting mixture was stirred for 15 minutes. 6 ml of the catalyst solution prepared in example 1 (2 mmol of Ti-catalyst) was added at 70°C. After addition of the catalyst the mixture was stirred for an additional 15 minutes at 70°C. 1.56 ml (22.7 mmol) of thioacetic acid was dosed to the reaction mixture at 70°C within 15 minutes. The reaction mixture was stirred for 1 hour at 70°C for completion of the conversion. The *e.e.* of (S)-3-thioacetyl-2-methylpropionic acid in the reaction mixture, as determined by HPLC, was 53 %.

### Example VI: preparation of enantiomerically enriched (S)-3-thioacetyl-2-methyl propionic acid

To 95 ml of toluene, 156 mg (1.44 mmol) of sodium methacrylate and 1.72 g (20 mmol) of methacrylic acid were added at 70°C. The resulting mixture was stirred for 15 minutes. 12 ml of the catalyst solution prepared in example 1 (4 mmol Ti-catalyst) was added at 70°C. After addition of the catalyst the mixture was stirred for an additional 15 minutes at 70°C. 1.56 ml (22.7 mmol) of thioacetic acid was dosed to the reaction mixture at 70°C within 15 minutes. The reaction mixture was stirred for 1 hour at 70°C to complete the conversion. The *e.e.* of (S)-3-thioacetyl-2-methylpropionic acid in the reaction mixture, as determined by HPLC, was 48%.

### Example VII: preparation of enantiomerically enriched (S)-2-(acetylthiomethyl)butyric acid

To 100 ml of toluene, 90 mg (0.83 mmol) of sodium methacrylate and 2.00 g (20 mmol) of 2-ethylacrylic acid were added at 70°C. The resulting mixture was stirred for 15 minutes. 8.5 ml of the catalyst solution prepared in example 1 (2.8 mmol Ti-catalyst) was added at 70°C. After addition of the catalyst the mixture was stirred for an additional 15 minutes at 70°C. 1.56 ml (22.7 mmol) of thioacetic acid was dosed to the reaction mixture at 70°C within 15 minutes. The reaction mixture was stirred for 1 hour at 70°C to complete the conversion. The *e.e.* of (S)-2-(acetylthiomethyl)butyric acid in the reaction mixture, as determined by HPLC, was 63%.

### Example VIII: preparation of enantiomerically enriched (S)-2-(acetylthiomethyl)hexanoic acid

To 100 ml of toluene, 104 mg (0.96 mmol) of sodium methacrylate and 2.75 g (21.5 mmol) of 2-butylacrylic acid were added at 70°C. The resulting mixture was stirred for 15 minutes. 9 ml of the catalyst solution prepared in example 1 (3 mmol Ti-catalyst) was added at 70°C. After addition of the catalyst the mixture was stirred for an additional 15 minutes at 70°C. 1.56 ml (22.7 mmol) of thioacetic acid was dosed to the reaction mixture at 70°C within 15 minutes. The reaction mixture was stirred for 1 hour at 70°C to complete the conversion. The *e.e.* of (S)-2-(acetylthiomethyl)hexanoic acid in the reaction mixture, as determined by HPLC, was 69 %.

### Example IX: preparation of enantiomerically enriched (S)-3-thioacetyl-2-methylproprionic acid

To 100 ml of toluene, 180 mg (1.67 mmol) of sodium methacrylate and 8.60 g (100 mmol) of methacrylic acid were added at 23°C. The resulting mixture was stirred for 15 minutes at 23°C. 15 ml of the catalyst solution prepared in example 1 was added at 23°C, followed by stirring the mixture for an additional 15 minutes at 23°C. 7.6 g (100 mmol) of thioacetic acid was added in 15 minutes and the mixture was stirred for 72 hours at 23°C. The conversion was > 90% according to HPLC. The *e.e.,* as determined by HPLC, was 23%.

### Example X: preparation of enantiomerically enriched (S)-3-thiobenzoyl-2-methylpropionic acid

To 100 ml of toluene, 180 mg (1.67 mmol) of sodium methacrylate and 8.60 g (100 mmol) of methacrylic acid were added at 70°C. The resulting mixture was stirred for 15 minutes at 70°C. 15 ml of the catalyst solution prepared in example 1 was added at 70°C, followed by stirring the mixture for an additional 15 minutes at 70°C. A solution of 13.8 g (100 mmol) of thiobenzoic acid in 50 ml of toluene was added in 18 minutes, after which the mixture was stirred for 3 hours at 70°C. The conversion was > 90% according to HPLC. The *e.e.,* as determined by HPLC, was 35%.

### Example XI: preparation of enantiomerically enriched (S)-3-thioacetyl-2-methylproprionic acid

To 100 ml of toluene, 180 mg (1.67 mmol) of sodium methacrylate and 8.60 g (100 mmol) of methacrylic acid were added at 70°C. The resulting mixture was stirred for 15 minutes at 70°C. 15 ml of the catalyst solution prepared in example 1 was added at 70°C, followed by stirring the mixture for an additional 15 minutes at 70°C. 7.6 g (100 mmol) of thioacetic acid was added in 15 minutes and the mixture was stirred for 3 hours at 70°C. The conversion was > 90% according to HPLC. The *e.e.,* as determined by HPLC, was 45%.

### Example XII: preparation of enantiomerically enriched (S)-3-thioacetyl-2-phenyl propionic acid

To 100 ml of toluene, 110 mg (1.0 mmol) of sodium methacrylate and 2.96 g (20 mmol) of 2-phenylacrylic acid were added at 70°C. The resulting mixture was stirred for 15 minutes at 70°C. 9 ml of the catalyst solution prepared in example 1 was added at 70°C, followed by strirring the mixture for an additional 15 minutes at 70°C. 1.52 grams of thioacetic acid (20 mmol) was added in 15 minutes, after which the mixture was stirred for 0.5 hours at 70°C. The *e.e.,* as determined by HPLC, was 48%.

### Example XIII: preparation of enantiomerically enriched (S)-3-thiobenzoyl-2-phenyl propionic acid

To 100 ml of toluene, 110 mg (1.0 mmol) of sodium methacrylate and 2.98 g (20 mmol) of 2-phenylacrylic acid were added at 70°C. The resulting mixture was stirred for 15 minutes at 70°C. 9 ml of the catalyst solution prepared in example 1 was added at 70°C, followed by stirring the mixture for an additional 15 minutes at 70°C. 2.60 g (20 mmol) of thiobenzoic acid was added in 15 minutes, after which the mixture was stirred for 0.5 hours at 70°C. The *e.e.,* as determined by HPLC, was 38%.

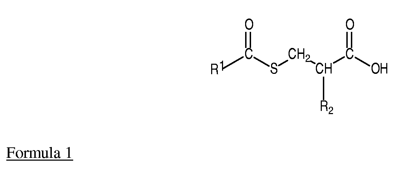

## Claims

1. Process for the preparation of an enantiomerically enriched compound of formula 1 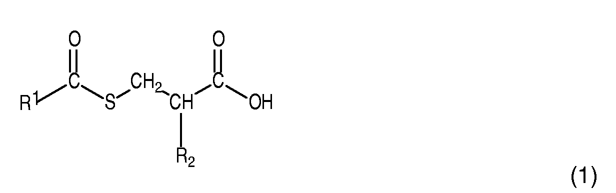 or a salt thereof, wherein R¹ and R² each independently represent a (hetero)alkyl or (hetero)aryl group, wherein a thio compound of formula 2 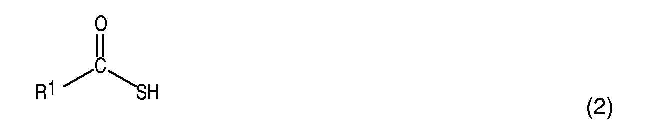 with R¹ as defined above, is reacted with an alkene of formula 3 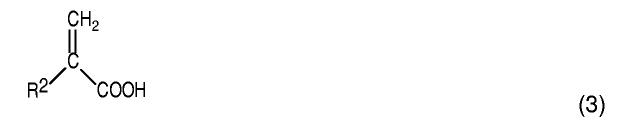 or a salt thereof, with R² as defined above, **characterized in that** the reaction takes place in the presence of an enantiomerically enriched Lewis acid containing a transition metal chosen from the group of Ti, Zr and Hf or a combination thereof and at least one enantiomerically enriched ligand, and in the presence of an alkali metal ion, an alkaline earth metal ion or an ammonium ion.

2. Process according to claim 1, wherein the transition metal is Ti.

3. Process according to claim 1 or 2, wherein the ligand is an enantiomerically enriched tartaric acid ester.

4. Process according to any one of claims 1-3, wherein the alkali metal ion, the alkaline earth metal ion or the ammonium ion is Na⁺ or Li⁺.

5. Process according any one of claims 1-4, wherein the R¹ represents a methyl group.

6. Process according to any one of claims 1-5, wherein R² represents a methyl group or a benzyl group.

7. Process according to any one of claims 1-6, **characterised in that** the molar ratio between R¹-SH and R²-C(=CH2)-C(O)OH in the reaction mixture is between 2.5:1 and 1:2.5.

8. Process according to any one of claims 1-7, **characterised in that** the enantiomerically enriched thio compound is isolated by contacting said compound with an amine and crystallisation of the salt formed.
